# EUROPEAN PATENT SPECIFICATION

(11) **EP 1 554 972 B1**
(45) Date of publication and mention of the grant of the patent: **11.04.2018**
(21) Application number: 03758757.3
(22) Date of filing: 22.10.2003
(51) Int. Cl.: A61B 1/005, A61B 1/31, A61B 1/00

(54) **ENDOSCOPE DEVICE**
ENDOSKOPVORRICHTUNG
DISPOSITIF ENDOSCOPIQUE

(30) Priority: 25.10.2002 JP 2002311598
(43) Date of publication of application: 20.07.2005
(73) Proprietor: Olympus Corporation, Tokyo 192-8507 (JP)
(72) Inventor: MORIYAMA, Hiroki, Akishima-shi, Tokyo 196-0032 (JP)
(74) Representative: von Hellfeld, Axel
(86) International application number: PCT/JP2003/013463
(87) International publication number: WO 2004/037075

(56) References cited:
- JP-A- 9 276 244
- JP-A- 11 042 206
- JP-A- 2000 137 172
- JP-A- 2001 029 313
- JP-A- 2001 070 241
- US-A- 5 885 208

## Description

### Technical Field

The present invention relates to an endoscope system in which the ease of insertion of an endoscope into the large intestine can be improved.

### Background Art

In general, the insertion unit of an endoscope includes a soft section having flexibility. A distal section is formed distally to the soft section with a bending section between them. An observation window, an illumination window, a treatment instrument passage channel opening, and others are arranged in a predetermined manner on the distal plane of the distal section.

As already known, the portion of the soft section on the distal side thereof is made more flexible so that it will be more soft and bendable. On the other hand, the portion of the soft section on the proximal side thereof is made little flexible so that the ease of insertion thereof will improve.

For example, Japanese Unexamined Patent Application Publication No. 2001-190494 has disclosed the technology of ensuring the flexibility of the portion of a soft section on the distal side thereof and improving the ease of insertion thereof. According to the technology, the outer diameter of the soft section is gradually increased from the distal end thereof to the proximal end thereof so that the flexibility of the portion of the soft section on the proximal side thereof will be smaller than that of the distal-side portion thereof.

However, assuming that one operator uses a plurality of endoscopes that have different capabilities and that are included in one endoscope system, if the outer diameters of the large-diameter portions of the soft sections of the respective endoscopes are largely different from one another, the operator will have a feeling of wrongness when holding the large-diameter portions of the respective endoscopes. Thus, insertion efficiency is impaired.

The present invention attempts to break through the above situation. An object of the present invention is to provide an endoscope system that does not give a feeling of wrongness to an operator when the operator holds the large-diameter portions of the soft sections of a plurality of endoscopes included in the endoscope system.

JP 2001 070241 A relates to an image processing device, wherein an extension circuit board for color processing, an extension circuit board for a static image and a circuit board for compressing/ recording a static image are piled in order and connected to an extension connector mounted on a main circuit board when this image processing device is used, for example, for the otorhinological treatment, and a data bus and an address bus of the CPU mounted on the main circuit board are connected to each extension circuit board. Various synchronous signals such as a clock (CLK), a horizontal synchronous signal (HD), a vertical synchronous signal (VD), a field judging signal (FLD), a complex synchronous signal (CSYNC), etc., are outputted from the SSG.

JP 2000 137172 A refers to an image pickup device consisting, successively from the object side, of a negative lens, a positive lens, a stop, a positive lens, cover glass and a CCD, and a reflection type filter is arranged between the negative lens and the positive lens and an absorption type filter is arranged between the stop and the positive lens. The overall characteristics at the time of combining the optical elements are set to satisfy the condition equation 0.45 ≤ |ΔT/ΔW| ≤ 0.75. In this equation, ΔT is the difference between the transmittance T(600) at a wavelength 600 nm and the transmittance T(550) at a wavelength 550 nm, i.e., ΔT=T(600)-T(550) and ΔW is 50 nm.

JP 2001 029313 A relates to an endoscope arranged with a CCD at the front end of an insertion part, which is freely attachably and detachably connected to a processor, by which the information previously stored in a ROM is transmitted to a control means in the processor. This control means controls the sensitivity of the CCD according to the connected endoscope by a CCD sensitivity control means so that the observation images of the adequate brightness may be obtained without depending upon the kinds of the endoscopes.

US 5,885,208 refers to an endoscope system having a first endoscope in which a hardness variation mechanism for use in adjusting the hardness level of a soft part of an insertion unit is incorporated in the soft part, and at least one second endoscope having a soft part. A range of levels of the adjustable hardness of the soft part of the first endoscope includes the hardness level of the soft part of the second endoscope. Another endoscope system has a first endoscope in which a hardness variation mechanism for use in adjusting the hardness level of a soft part of an insertion unit is incorporated in the soft part, and at least one second endoscope usable for examination of the same region as the first endoscope and having a soft part. A range of levels of the adjustable hardness of the soft part of the first endoscope includes the hardness level of the soft part of the second endoscope.

JP 9276244 relates to an MR endoscope, having a nuclear magnetic resonance signal detecting coil built into the end of an inserted part, which is provided with the long inserted part for insertion into a celom, an operation part for operating the inserted part at hand, and a soft connection for connecting the inserted part to the operation part, the connection being large in diameter and made higher in rigidity than the inserted part.

JP 11 042 206 A refers to an endoscope system at least having a first endoscope provided with a hardness controlling mechanism for controlling the hardness of a flexible part and a second endoscope having the same object for use as the object for use of the first endoscope. In this case, the constitution is made in such a way that the hardness of the flexible part of the second endoscope is within the range for controlling the hardness of the flexible part of the first endoscope and, in case of using properly each of the endoscopes, it is possible for an operator to decrease as much as possible a sence of incongruity on inserting properties and to be convenient for use.

### Disclosure of Invention

The present invention provides an endoscope system according to the appended claims.

### Brief Description of the Drawings

Fig. 1 to Fig. 7D show a first example, not forming part of the present invention;
Fig. 1 shows the overall configuration of an endoscope;
Fig. 2 is an enlarged view of the distal side of an endoscope insertion unit;
Fig. 3 is an enlarged sectional view showing the internal structure of a soft section that is a major portion;
Fig. 4A to Fig. 4C are explanatory diagrams showing a process of molding a flexible tube that sheathes the soft section;
Fig. 5A to Fig. 5D are explanatory diagrams showing a process of molding the flexible tube that sheathes the soft section according to a variant;
Fig. 6 is an explanatory diagram showing indices printed on the surface of the flexible tube;
Fig. 7A to Fig. 7D are explanatory diagrams showing the states of the insertion unit of an endoscope inserted into the large intestine;
Fig. 8 schematically shows the configuration of an endoscope system in accordance with an embodiment of the present invention; and
Fig. 9 is an enlarged view of the distal part of an endoscope included in a further example.

### Best Mode for Carrying Out the Invention

Referring to drawings, embodiments of the present invention and examples (not forming part of the claimed invention) that are useful for understanding the present invention will be described below.

### (first example)

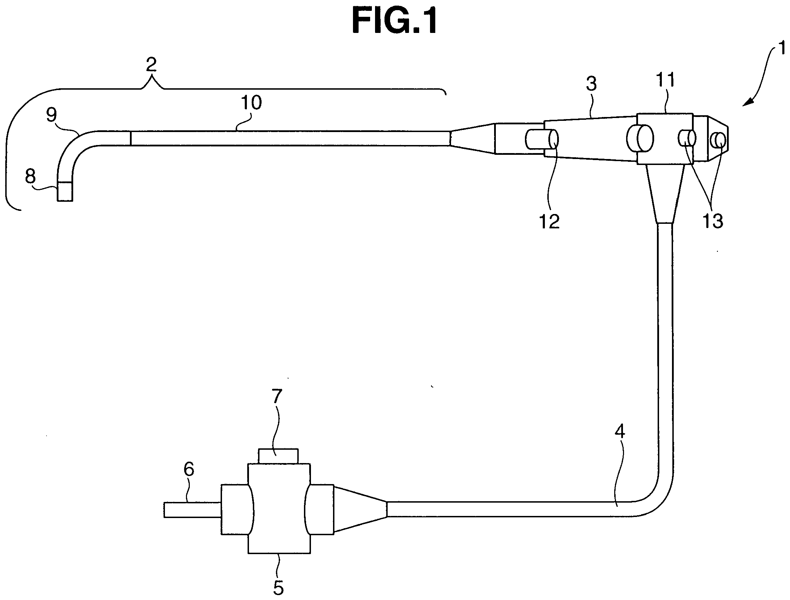

Fig. 1 to Fig. 7D show a first example, not forming part of the present invention.

As shown in Fig. 1, an endoscope 1 includes: an insertion unit 2 having a solid-state imaging device such as a CCD incorporated in the distal part thereof; an operating unit 3 coupled to the proximal end of the insertion unit 2 and held and handled by an observer; and a universal cord 4 extended from the operating unit 3.

A connector unit 5 is formed at the end of the universal cord 4. The connector unit 5 includes a light guide connector 6 and a camera connector 7. The light guide connector 6 and camera connector 7 are coupled to peripheral equipment including a light source unit and a camera control unit.

The insertion unit 2 has, from the distal end thereof, a distal section 8, a bending section 9 that can be freely bent, and a soft section 10 having flexibility. The proximal end of the soft section 10 is coupled to the operating unit 3. Incidentally, an observation window, an illumination window, a treatment instrument passage channel opening, and an aeration/perfusion nozzle are arranged in a predetermined manner on the distal plane of the distal section 8. These are not shown in the drawing.

On the other hand, the operating unit 3 includes an angulation lever 11 used to remotely control bending of the bending section 9, a treatment instrument insertion port 12 through which a treatment instrument such as forceps are inserted, and a plurality of switches 13 used to freeze or expose an image.

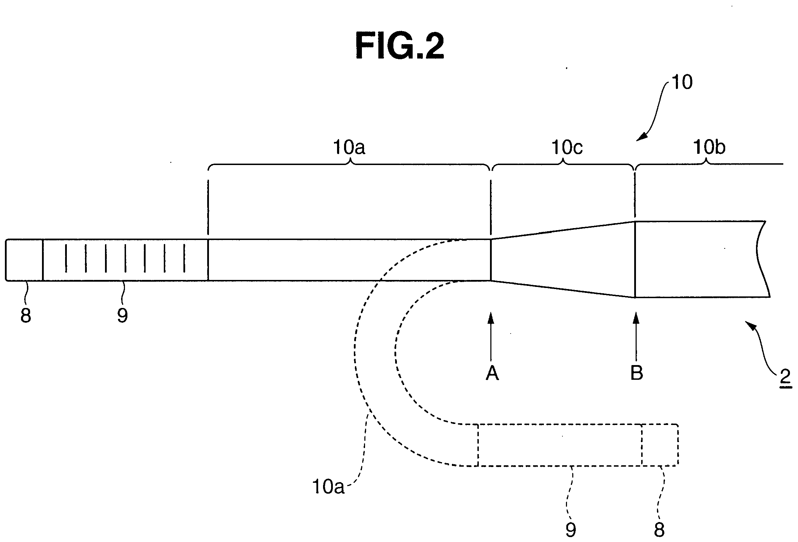

As shown in Fig. 2, the soft section 10 has: a small-diameter portion 10a which is formed on the distal side of the soft section and whose outer diameter is substantially the same over the whole length thereof; a large-diameter portion 10b which is formed on the operator side of the soft section opposite to the small-diameter portion 10a and whose outer diameter is larger than the outer diameter of the small-diameter portion 10a; and a tapered portion 10c that smoothly links the small-diameter portion 10a and large-diameter portion 10b. In Fig. 2, the border between the small-diameter portion 10a and tapered portion 10c is indicated with arrow A, and the border between the tapered portion 10c and large-diameter portion 10b is indicated with arrow B.

The length from the distal end of the distal section 8 that is the distal part of the insertion unit 2 to the rear end of the small-diameter portion 10a indicated with arrow A may be 30 cm, 40 cm, or 50 cm, or in other words, varies depending on the purpose of use of the endoscope 1. Normally, an endoscope having the length of 70 cm does not exist. Consequently, the rear end of the small-diameter portion 10a indicated with arrow A is located forward a point that is separated from the distal end of the distal section 8 by 70 cm.

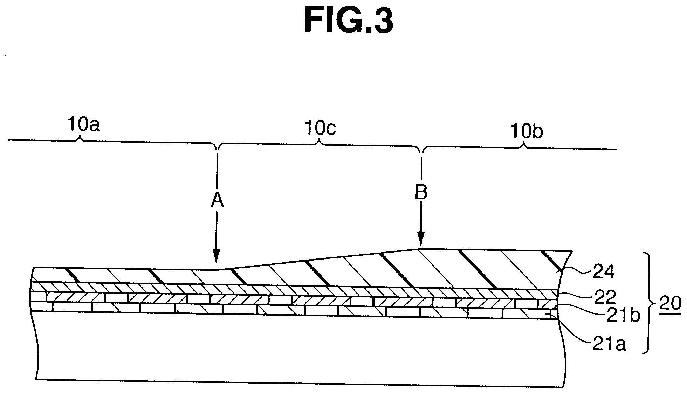

Fig. 3 schematically shows part of the internal structure of the soft section 10.

A flexible tube 20 that is an armor of the soft section 10 comprises, from the internal side thereof, spiral tubes 21a and 21b that wind in mutually opposite directions, a braided tube 22, and a sheathing resin 24. The spiral tubes 21a and 21b and braided tube 22 have substantially constant inner and outer diameters over the whole lengths thereof. The thickness of the sheathing resin 24 is varied in order to form the small-diameter portion 10a, large-diameter portion 10b, and tapered portion 10c linking the small-diameter and large-diameter portions. According to the present embodiment, two layers of spiral tubes are included as the spiral tubes 21a and 21b. Alternatively, the number of layers of spiral tubes may be one or three or more.

A method of molding the flexible tube 20 is, for example, such that the spiral tubes 21a and 21b and braided tube 22 which are assembled in a predetermined manner are used as a core die to perform extrusion molding so that the sheathing resin 24 will mold to the braided tube 22. At this time, the small-diameter portion 10a, tapered portion 10c, and large-diameter portion 10b are formed by changing the speed at which the core die is pulled out.

To be more specific, the speed at which the core die is pulled out is raised in order to form the small-diameter portion 10a, and lowered in order to form the large-diameter portion 10b. While molding proceeds from the stage in which the small-diameter portion 10a is formed to the stage in which the large-diameter portion 10b is formed or vice versa, the pullout speed is changed continuously in order to form the tapered portion 10c.

However, the above molding method employs dies having the same inner diameter. Therefore, it is hard to form the portions 10a to 10c having precise outer diameters.

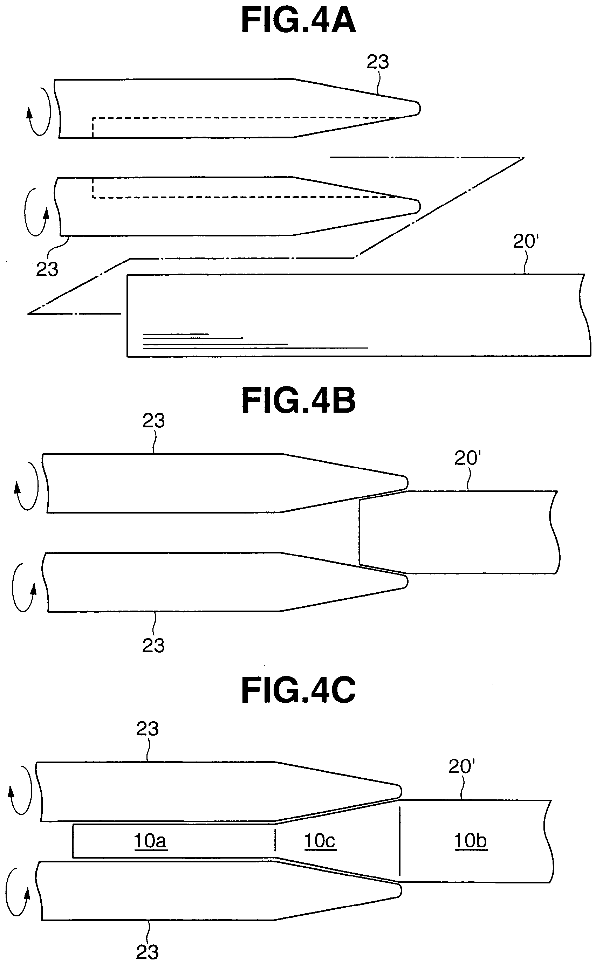

In contrast, as shown in Fig. 4A to Fig. 4C, a plurality of tapered grinding stones 23 is used to perform centerless grinding on a bar-like flexible tube material 20' manufactured to have a sole outer diameter. If the small-diameter portion 10a and tapered portion 10c are thus formed, the portions 10a to 10c will have precise outer diameters.

Specifically, as shown in Fig. 4A, the flexible tube material 20' having the same outer diameter as the large-diameter portion 10b is relatively approached to the axial core of the plurality of tapered grinding stones 23 arranged circumferentially around the flexible tube material 20'. Thereafter, as shown in Fig. 4B, the flexible tube material 20' is pressed against the plurality of tapered grinding stones 23. The flexible tube material 20' thus has the surface thereof ground in line with the shape defined with the plurality of tapered grinding stones 23. Consequently, the distal part of the flexible tube material 20' has the small-diameter portion 10a and tapered portion 10c formed as shown in Fig. 4C.

According to the centerless grinding, the small-diameter portion 10a, tapered portion 10c, and large-diameter portion 10b can be formed highly precisely.

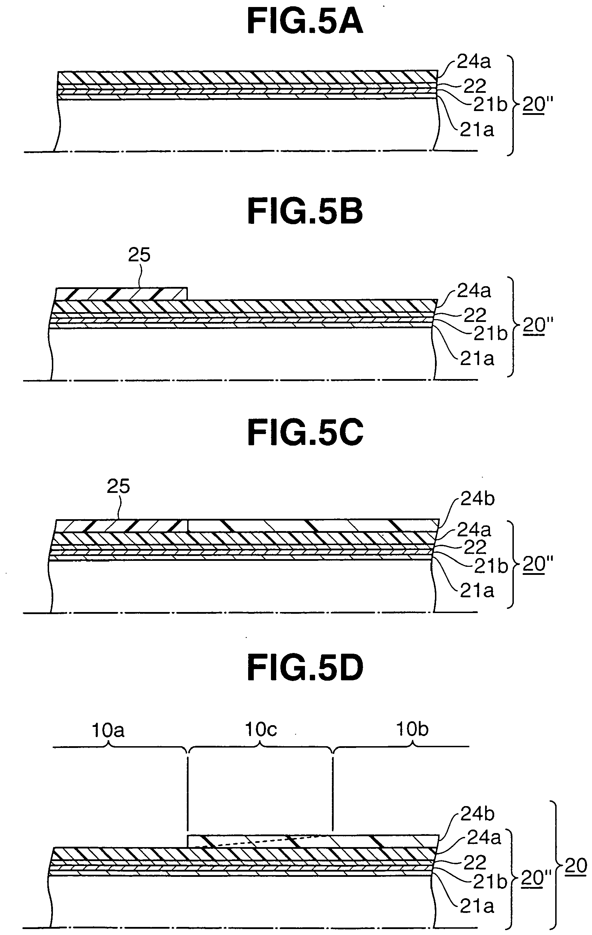

Otherwise, the small-diameter portion 10a, tapered portion 10c, and large-diameter portion 10b may be formed highly precisely by performing extrusion molding as shown in Fig. 5A to Fig. 5D.

Specifically, according to this molding method, first, as shown in Fig. 5A, a flexible tube material 20" having the same outer diameter as the small-diameter portion 10a over the whole length thereof and comprising the spiral tubes 21a and 21b, the braided tube 22, and a first sheathing resin 24a is manufactured by performing extrusion molding. Thereafter, as shown in Fig. 5B, the portion of the flexible tube material 20" corresponding to the small-diameter portion 10a is sheathed with a tube member 25 such as a heat contractile tube. In this state, as shown in Fig. 5C, different dies are used to perform extrusion molding again. Thus, a second sheathing resin 24b having the same outer diameter as the large-diameter portion 10b is formed around the periphery of the flexible tube material 20" successively to the tube member 25.

Thereafter, as shown in Fig. 5D, the tube member 25 is peeled off from the flexible tube material 20". The portion of the second sheathing resin 24b corresponding to the tapered portion 10c is, as indicated with a dashed line, ground using a grinder or the like. This results in the flexible tube 20 having the small-diameter portion 10a, tapered portion 10c, and large-diameter portion 10b as shown in Fig. 2 or Fig. 3.

Thereafter, the flexible tube 20 is heated in order to thermally weld the first sheathing resin 24a, second sheathing resin 24b, and braided tube 22. Thus, the respective members are firmly bonded to one another.

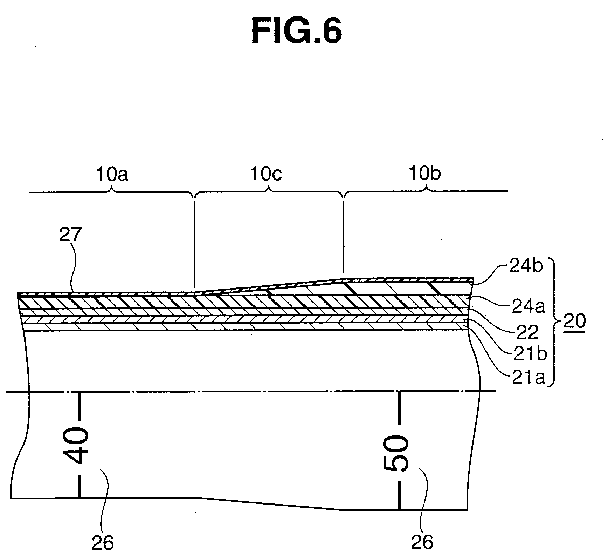

In this case, as shown in Fig. 6, in the next process, indices 26 may be printed on the surface of the flexible tube 20, and a thin top coat 27 may be coated over the indices 26. By coating the indices 26 with the top coat 27, the border between the first sheathing resin 24a and second sheathing resin 24b can be further smoothed.

Referring to Fig. 6, the tapered portion 10c is interposed between two of the indices 26 printed equidistantly over substantially the whole length of the soft section 10. The indices 26 indicate, according to the present embodiment, distances from the distal end of the distal section 8 of the endoscope. "40" signifies that the point is separated 40 cm from the distal end, and "50" signifies that the point is separated 50 cm from the distal end.

According to the present example, not forming part of the claimed invention, the tapered portion 10c is formed to extend from a point separated approximately 43 cm from the distal end to a point separated approximately 48 cm therefrom. When the tapered portion 10c is interposed between two of the plurality of indices 26 printed equidistantly, the index 26 need not be printed on the tapered portion 10c. Since it is labor-intensive to print an index on the tapered portion 10c, when it is unnecessary to print an index thereon, the cost of manufacture is reduced.

Incidentally, the small-diameter portion 10a is long enough to bend 180° or more as indicated with a dashed line in Fig. 2 when the small-diameter portion 10a is bent to form an arc having a minimum radius. When it says that the small-diameter portion 10a is bent to form an arc having a minimum radius, it means that the small-diameter portion 10a is bent naturally to such an extent that the belt-like wire made into either of the spiral tubes 21a and 21b is folded, and that the small-diameter portion 10a cannot be bent further.

Next, the operation of the present example having the foregoing components will be described below.

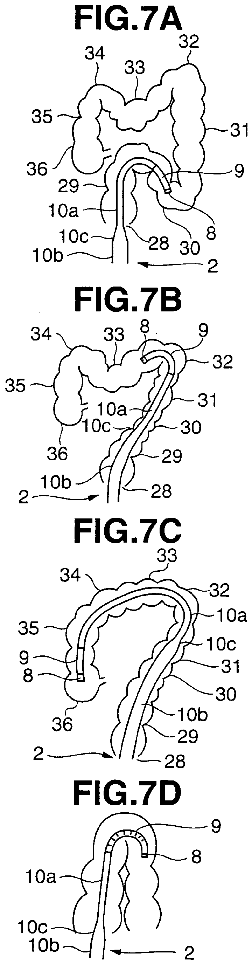

Fig. 7A to Fig. 7D show states in which the insertion unit 2 of the endoscope 1 included in the present example, not forming part of the claimed invention, is inserted into the large intestine. The large intestine mainly comprises the anus 28, rectum 29, sigmoid colon 30, descending colon 31, splenic curvature 32, transverse colon 33, hepatic curvature 34, ascending colon 35, and appendix 36.

Fig. 7A shows the state in which the insertion unit 2 of the endoscope 1 is inserted into the sigmoid colon 30. In general, the sigmoid colon 30 is the most complexly tortuous among all the parts of the large intestine. Moreover, the sigmoid colon 30 is soft and movable. When the insertion unit 2 is inserted while moved in line with the shape of the sigmoid colon 30, the distal side of the soft section 10 should be bent a little as softly as possible. When the insertion unit 2 must be inserted into the sigmoid colon 30 or any other complexly tortuous region, if the soft section 10 has the small-diameter portion 10a as the distal side thereof like the one of the endoscope 1 included in the present embodiment, the insertion unit 2 can be smoothly introduced into the sigmoid colon 30 or any other complexly tortuous region.

As shown in Fig. 7B, when the distal section 8 of the insertion unit 2 passes through the descending colon 31 and enters the splenic curvature 32, the insertion unit 2 is pulled once. Thus, the sigmoid colon 30 that is soft and movable is folded and shortened, and substantially straightened.

With the sigmoid colon 30 shortened and straightened as mentioned above, the distal section 8 of the insertion unit 2 is advanced further deeply beyond the splenic curvature 32. When the distal section 8 of the insertion unit 2 is advanced deeply, the insertion unit 2 entirely moves in the direction of advancement and the soft section 10 warps. Consequently, the sigmoid colon 30 that is shortened and straightened is restored to the original tortuous shape.

Owing to the restoration force of the sigmoid colon 30, the operator's handling is hardly conveyed to the distal section 8. The ease of insertion of the insertion unit 2 is degraded. Therefore, the portion of the soft section 10 inserted into the large intestine in the state shown in Fig. 7B should be relatively thick and hard because it hardly warps and is therefore easily handled. Consequently, as long as at least part of the tapered portion 10c interposed between the small-diameter portion 10a formed on the distal side of the soft section 10 and the large-diameter portion 10b formed on the operator side thereof is inserted into the large intestine, since the outer diameter of the soft portion 10 gradually increases from the tapered portion, the operator's handling performed near, for example, the anus 28 is easily conveyed to the distal section 8 of the insertion unit 2. This leads to improved maneuverability.

In this case, as shown in Fig. 7B, the distal section 8 of the endoscope enters the splenic curvature 32. Consequently, the sigmoid colon 30 is shortened and straightened. The length from the point on the insertion unit located at the anus 28 to the distal section 8 of the endoscope ranges from approximately 40 cm to approximately 45 cm. This has been revealed in "Total Colonoscopy by One-man Handling: passage through bilateral colonic curvatures" (Endoscope for Examination of the Alimentary Tract, Vol. 5, No. 5, 1993, P.629-P.633). Therefore, as long as at least part of the tapered portion 10c is located forward the point separated from the distal end of the distal section 8 of the endoscope by 45 cm, the operator's handling performed near the anus 28 can be smoothly conveyed to the distal section 8 of the insertion unit 2.

As shown in Fig. 7C, when the distal section 8 of the endoscope reaches the appendix 36, not only the sigmoid colon 30 but also the transverse colon 33 is shortened and straightened. According to the above literature, when the insertion unit 2 is inserted the shortest distance into the appendix 36 by way of the anus 28, the portion of the soft section 10 located at the anus 28 is the portion thereof separated by approximately 60 cm to approximately 70 cm from the distal section 8 of the endoscope.

Therefore, the operator side of the soft section 10 is made thick so that it will hardly warp but can be twisted with less force and the operator's twisting will be easily conveyed to the distal section 8 of the endoscope. For this purpose, at least part of the tapered portion 10c must lie forward an endoscope portion separated 70 cm from the distal section 8 of the endoscope. Otherwise, the tapered portion 10c and large-diameter portion 10b would hardly enter the large intestine of a patient. An expected advantage would not be provided.

At least part of the tapered portion 10c is disposed forward an endoscope portion separated 70 cm from the distal end of the distal section 8 of the endoscope. Consequently, when the distal section 8 of the endoscope is inserted deeply into the large intestines of almost all patients, the tapered portion 10c and large-diameter portion 10b can be invaded into the shortened sigmoid colon 30. The soft section 10 will therefore hardly warp and can prevent the sigmoid colon 30 from being restored to the original shape. Moreover, the operator puts his/her hand on the tapered portion 10c or large-diameter portion 10b of the soft section 10. The operator can therefore easily twist the insertion unit, and the twisting will be effectively conveyed to the distal section 8 of the endoscope.

As mentioned above, at least part of the tapered portion 10c linking the small-diameter portion 10a that is formed on the distal side of the soft section 10 and the large-diameter portion 10 that is formed on the operator side thereof is disposed forward an endoscope portion separated by 45 cm or 70 cm from the distal end of the distal section 8 of the endoscope. Anyhow, the position of the tapered portion 10 is determined optimally. Thus, the maneuverability in inserting the endoscope can be improved.

Fig. 7D shows a state in which part of the sigmoid colon 30 is bent acutely. As seen from Fig. 7D, when the sigmoid colon is bent so acutely that the colonic wall will be folded (or conglutinated), the angle of the curvature is as large as approximately 180° at maximum.

In order to pass the distal section 8 of the endoscope and succeeding bending section 9 through the region bent most acutely, the bending section 9 is requested to be bendable about 180°. Moreover, the small-diameter portion 10a that is the distal side of the soft section 10 adjoining the bending section 9 is requested to be naturally bendable on receipt of extraneous force up to 180°.

According to the present example, not forming part of the claimed invention, the small-diameter portion is long enough to be bent 180° when it is bent to form an arc having the smallest radius. Therefore, the endoscope can be relatively easily passed through the most acute curvature of the large intestine.

In this case, since the tapered portion 10c and large-diameter portion 10b are harder than the small-diameter portion 10a, if the small-diameter portion 10a is too short, the soft section 10 cannot be bent up to 180°. It is hard to pass the soft section 10 through an acute curvature of the large intestine.

Consequently, the relationship among the small-diameter portion 10a, tapered portion 10c, and large-diameter portion 10b in terms of the length in an axial direction is determined appropriately. Consequently, the soft section 10 can be passed through even the most acute curvature. Eventually, the ease of insertion of the endoscope insertion unit 2 improves.

### (Embodiment)

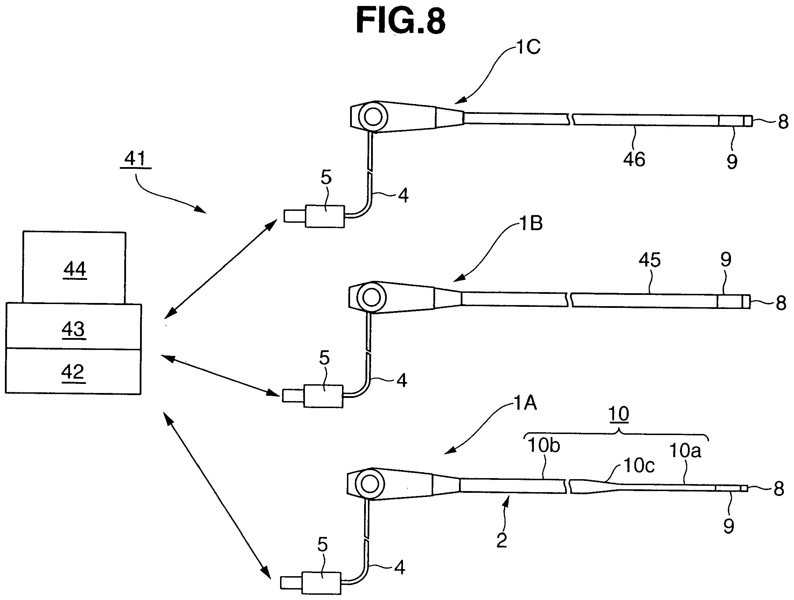

Fig. 8 shows an embodiment of the present invention. As illustrated, an endoscope system 41 in accordance with the present embodiment comprises a plurality of endoscopes 1A, 1B, and 1C that has different capabilities, and a light source unit 42, a video processor 43, and a monitor 44 which can be connected in common to the endoscopes 1A, 1B, and 1C. Each of the endoscopes 1A, 1B, and 1C has a connector unit 5 formed at the tip of a universal cord 4 extending from an operating unit thereof. The connector unit 5 is joined selectively to the light source unit 42 and video processor 43. In the drawing, three types of endoscopes 1A, 1B, and 1C are shown. Alternatively, four or more types of endoscopes may be included.

The first endoscope 1A has the same capabilities as the endoscope 1 included in the first example The soft section 10 of the first endoscope 1A comprises the small-diameter portion 10a, the large-diameter portion 10b, and the tapered portion 10c linking the small-diameter portion 10a and large-diameter portion 10b. Soft sections 45 and 46 of the second and third endoscopes 1B and 1C respectively have substantially the same outer diameters over the whole lengths thereof. The soft section 45 of the second endoscope 1B has a relatively large outer diameter, while the soft section 46 of the third endoscope 1C has a relatively small outer diameter.

In this case, the outer diameter of at least the large-diameter portion 10b of the soft section 10 of the first endoscope 1A is substantially equal to (with ± 5% or less) or smaller than the outer diameter of the soft section 45 of the second endoscope 1B. Moreover, the outer diameter of the large-diameter portion 10b of the first endoscope 1A is substantially equal to (with ± 5% or less) or larger than the outer diameter of the soft section 46 of the third endoscope 1C. In short, the outer diameter of the large-diameter portion 10b ranges from the outer diameter of the soft section 45 of the second endoscope 1B to the outer diameter of the soft section 46 of the third endoscope 1C.

Incidentally, the outer diameter of the small-diameter portion 10a of the soft section 10 of the first endoscope 1A may be substantially equal to the outer diameter of the soft section 46 of the third endoscope 1C. The outer diameter of the large-diameter portion 10b may be substantially equal to the outer diameter of the soft section 45 of the second endoscope 1B.

Next, the operation of the present embodiment having the foregoing components will be described below.

Inserting the insertion unit 2 of the first endoscope 1A into the large intestine is identical to that performed in the first embodiment. The description will therefore be omitted.

Whether the insertion unit 2 of the first endoscope 1A can be smoothly inserted into the large intestine depends on the structure of the insertion unit 2 itself. Moreover, it is essential that an operator should be less fatigued with the insertion.

In general, when an operator handles a colonoscope (for example, the first endoscope 1A), the operator holds the insertion unit 2 with his/her right hand and the operating unit 3 with his/her left hand. By moving the right and left hands harmoniously, the operator performs insertion, observation, and treatment. The operator thrusts, pulls, or twists the insertion unit 2 with the right hand. Generally, when the soft section 10 has a large outer diameter, the soft section 10 can be twisted with less force. The operator will therefore be less fatigued.

On the other hand, each operator is accustomed to the outer diameter of the soft section 10. Even when it says that the larger outer diameter of the soft section 10 will less fatigue an operator, if the operator has to handle the very thick soft section 10 with which he/she is unaccustomed, the operator would feel that something is uncomfortable and be fatigued.

According to the present embodiment, the outer diameter of the large-diameter portion 10b of the first endoscope 1A ranges from the largest outer diameter of the soft sections 45 and 46 of the second and third endoscopes 1B and 1C to the smallest outer diameter thereof. An operator will hardly feel that something is uncomfortable when handling the first endoscope 1A. Preferably, the outer diameter of the large-diameter portion 10b of the first endoscope 1A is made substantially equal to the outer diameter of the soft section 45 of the second endoscope 1B. In this case, an operator can handle the first endoscope 1A without a feeling that something is uncomfortable.

When the tapered portion 10c and large-diameter portion 10b of the soft section 10 of the first endoscope 1A occupies a half or more of the whole length of the insertion unit (effective length), an operator usually grasps the tapered portion 10c or large-diameter portion 10b. Therefore, if the outer diameter of the small-diameter portion 10a is smaller than the outer diameter of the soft section 46 of the third endoscope 1C, as long as the outer diameter of at least the tapered portion 10c or large-diameter portion 10b ranges from the outer diameter of the soft section 45 of the second endoscope 1B to the outer diameter of the soft section 46 of the third endoscope 1C, the operator's feeling that something is uncomfortable will be alleviated.

The operator grasps the small-diameter portion 10a in an early stage of insertion or a final stage of removal. Therefore, preferably, the outer diameter of the small-diameter portion 10a ranges from the outer diameter of the soft section 45 of the second endoscope 1B to the inner diameter of the ascending colon 35 (see Fig. 7). Furthermore, when the outer diameter of the small-diameter portion 10a is made substantially equal to the outer diameter of the soft section 46 of the third endoscope 1C, the operator can handle the small-diameter portion 10a without a feeling that something is uncomfortable.

As mentioned above, the present embodiment provides the same advantage as the first embodiment. In addition, the feeling that something is uncomfortable which an operator has while handling the insertion unit 2 of the first endoscope 1A can be largely alleviated.

### (Further example)

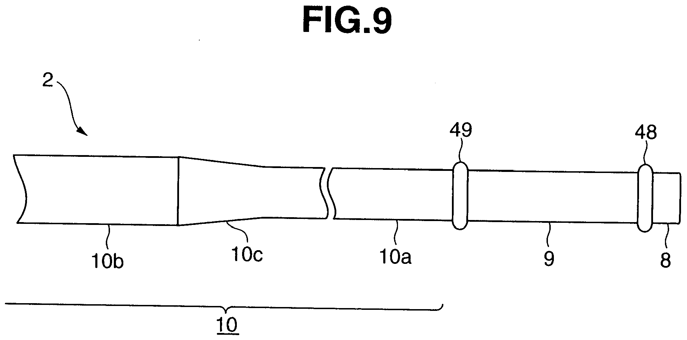

Fig. 9 shows a further example, not forming part of the present invention. The shape of an insertion unit is the same as that of the insertion unit 2 included in the first embodiment. The description of the insertion unit will therefore be omitted.

According to the example, at least one of connector sheathes 48 and 49 each linking portions is mounted on the border between the distal section 8 of the insertion unit 2 and the bending section 9 thereof or between the bending section 9 and the small-diameter portion 10a that is the distal side of the soft section 10. In the drawing, both the connector sheathes 48 and 49 are mounted. The connector sheathes 48 and 49 are formed with any of various kinds of members, such as, hard tubular members, an adhesive, or soft heat contractile tubes.

The endoscope included in the present example is designed so that the outer diameter of the large-diameter portion 10b that is the operator side of the soft section 10 will be equal to (with ± 5%) or slightly smaller than the outer diameter of the connector sheathes 48 and 49. For example, assuming that the outer diameter of the connector sheathes 48 and 49 is 12.8 mm, the outer diameter of the large-diameter portion 10b is set to 12.8 mm and the outer diameter of the small-diameter portion 10a is set to 11.5 mm.

Conventionally, the outer diameter of the connector sheathes 48 and 49 of an endoscope is generally larger than the outer diameter of the soft section 10. Therefore, when the endoscope is inserted into the large intestine, first, the lumen of the large intestine is observed using the distal section 8. If the distal section 8 and bending section 9 can be passed through a region in the large intestine, it will not be hard to pass the succeeding soft section 10 through the region.

However, this is not true when the outer diameter of the soft section 10 is apparently larger than the outer diameters of the large-diameter portion 10b and connector sheathes 48 and 49. Specifically, even when the distal section 8 and bending section 9 can be passed through a certain region in the large intestine, if the gap between the internal wall of the large intestine and the distal section 8 or bending section 9 is very narrow, it is impossible or hard to pass the succeeding large-diameter portion 10b through the region.

For example, when part of the sigmoid colon 30 shown in Fig. 7A is narrow, although the distal section 8 and bending section 9 can be passed through the part, it may be hard to pass the large-diameter section 10b through it. In this case, the insertion unit 2 cannot be smoothly advanced in the state shown in Fig. 7B. An operator may not find out the cause making it hard to advance the insertion unit 2 and may therefore not take proper measures.

According to the present example, the outer diameter of the large-diameter portion 10b is substantially equal to or smaller than the outer diameter of the connector sheathes 48 and 49. Therefore, if the connector sheathes 48 and 49 can be passed through a region, the large-diameter portion 10b can be passed through it. Consequently, when the insertion unit 2 cannot be smoothly advanced any longer, since the cause does not lie in the thickness of the large-diameter portion 10b, the real cause may be inferred in the same manner as it is in the conventional endoscope insertion unit 2.

As mentioned above, at least one of the connector sheathes 48 and 49 each linking portions is mounted on the border between the distal section 8 of the insertion unit 2 and the bending section 9 thereof or between the bending section 9 and the small-diameter portion 10a that is the distal side of the soft section 10. Moreover, the outer diameter of the large-diameter portion 10b that is the operator side of the soft section 10 is substantially equal to or slightly smaller than the outer diameter of the connector sheathes 48 and 49. Therefore, when the insertion unit 2 is inserted, the insertion will not be hindered because of the thickness of the large-diameter portion 10b. The great ease of insertion can be guaranteed.

### Industrial Applicability

As described so far, according to the present invention, an operator will not have a feeling of wrongness when holding the large-diameter portions of the soft sections of a plurality of endoscopes included in one endoscope system. Thus, excellent insertion efficiency is ensured.

## Claims

1. An endoscope system (41) comprising:
a first endoscope (1A) including a soft section (10) that has a small-diameter portion (10a) and a large-diameter portion (10b), wherein the small-diameter portion (10a) is a distal portion and the large-diameter portion (10b) is formed proximally to the small-diameter portion (10a) and whose outer diameter is larger than the outer diameter of the small-diameter portion (10a), the outer diameter of the larger diameter portion (10b) being substantially the same over the entire length thereof, the soft section (10) being included in an insertion unit;
a second endoscope (1B) which shares the same light source unit (42) or video processor (43) with the first endoscope (1A) and whose soft section (45) has substantially the same outer diameter over the whole length thereof, the soft section (45) being included in the insertion unit; and
a third endoscope (1C) which shares the same light source unit (42) or video processor (43) with the first endoscope (1A) and the second endoscope (1B) and whose soft section (46) has substantially the same outer diameter over the whole length thereof and has the outer diameter smaller than the outer diameter of the soft section (45) of the second endoscope (1B) the soft section (46) being provided in the insertion unit, wherein:
the outer diameter of the large-diameter portion (10b) of the soft section (10) of the first endoscope (1A) is substantially identical to or smaller than the outer diameter of the soft section (45) of the second endoscope (1B) and is larger than the outer diameter of the soft section (46) of the third endoscope (1C), and
the outer diameter of the small-diameter portion (10a) of the soft section (10) of the first endoscope (1A) is substantially identical to the outer diameter of the soft section (46) of the third endoscope (1C).

2. An endoscope system (41) according to Claim 1, wherein the outer diameter of the small-diameter portion (10a) of the first endoscope (1A) is larger than the outer diameter of the soft section (46) of the third endoscope (1C).

3. An endoscope system (41) according to Claim 1 or 2, wherein the outer diameter of the large-diameter portion (10b) of the first endoscope (1A) is substantially identical to the outer diameter of the soft-section (45) of the second endoscope (1B).

4. An endoscope system (41) according to any of Claims 1 to 3, wherein the soft section (10) of the first endoscope (1A) further includes a tapered portion (10c) linking the small-diameter portion (10a) and the large-diameter portion (10b).

## Patentansprüche

1. Endoskopsystem (41) aufweisend:
ein erstes Endoskop (1A) mit einem weichen Bereich (10), der einen Abschnitt (10a) mit einem kleinen Durchmesser und einen Abschnitt (10b) mit einem großen Durchmesser umfasst, wobei der Abschnitt (10a) mit einem kleinen Durchmesser ein distaler Abschnitt und der Abschnitt (10b) mit einem großen Durchmesser proximal zu dem Abschnitt (10a) mit kleinem Durchmesser ausgebildet ist, wobei der Außendurchmesser des Abschnitts (10b) mit großem Durchmesser größer als der Außendurchmesser des Abschnitts (10a) mit kleinem Durchmesser ist, wobei der Außendurchmesser des Abschnitts (10b) mit großem Durchmesser über die gesamte Länge des Abschnitts (10b) im wesentlichen gleich groß ist, wobei der weiche Bereich (10) von einer Einführeinheit umfasst ist;
ein zweites Endoskop (1B), das die gleiche Lichtquelleneinheit (42) oder den gleichen Videoprozessor (43) wie das erste Endoskop (1A) verwendet, und dessen weicher Bereich (45) im Wesentlichen den gleichen Außendurchmesser über seine gesamte Länge hat, wobei der weiche Bereich (45) von einer Einführeinheit umfasst ist;
ein drittes Endoskop (1C), das die gleiche Lichtquelleneinheit (42) oder den gleichen Videoprozessor (43) wie das erste Endoskop (1A) und das zweite Endoskop (1B) verwendet, und dessen weicher Bereich (46) im wesentlichen denselben Außendurchmesser über seine gesamte Länge hat und einen Außendurchmesser aufweist, der kleiner als der Außendurchmesser des weichen Bereichs (45) des zweiten Endoskops (1B) ist, wobei der weiche Bereich (46) in der Einführeinheit vorgesehen ist, wobei:
der Außendurchmesser des Abschnitts (10b) mit großem Durchmesser des weichen Bereichs (10) des ersten Endoskops (1A) im Wesentlichen identisch zu oder kleiner als der Außendurchmesser des weichen Bereichs (45) des zweiten Endoskops (1B) und größer als der Außendurchmesser des weichen Bereichs (46) des dritten Endoskops (1C) ist, und
der Außendurchmesser des Abschnitts (10a) mit kleinem Durchmesser des weichen Bereichs (10) des ersten Endoskops (1A) im Wesentlichen identisch zu dem Außendurchmesser des weichen Bereichs (46) des dritten Endoskops (1C) ist.

2. Endoskopsystem (41) nach Anspruch 1, wobei der Außendurchmesser des Abschnitts (10a) mit kleinem Durchmesser des ersten Endoskops (1A) größer als der Außendurchmesser des weichen Bereichs (46) des dritten Endoskops (1C) ist.

3. Endoskopsystem (41) nach Anspruch 1 oder 2, wobei der Außendurchmesser des Abschnitts (10b) mit großem Durchmesser des ersten Endoskops (1A) im Wesentlichen identisch zu dem Außendurchmesser des weichen Bereichs (45) des zweiten Endoskops (1B) ist.

4. Endoskopsystem (41) nach einem der Ansprüche 1 bis 3, wobei der weiche Bereich (10) des ersten Endoskops (1A) ferner einen konischen Abschnitt (10c) aufweist, der den Abschnitt (10a) mit kleinem Durchmesser und den Abschnitt (10b) mit großem Durchmesser verbindet.

## Revendications

1. Système (41) d'endoscope comprenant :
un premier endoscope (1A) incluant une section souple (10) qui présente une partie de petit diamètre (10a) et une partie de grand diamètre (10b), dans lequel la partie de petit diamètre (10a) est une partie distale et la partie de grand diamètre (10b) est formée de manière proximale par rapport à la partie de petit diamètre (10a) et dont le diamètre externe est plus grand que le diamètre externe de la partie de petit diamètre (10a), le diamètre externe de la partie de plus grand diamètre (10b) étant sensiblement le même sur la totalité de sa longueur, la section souple (10) étant contenue dans une unité d'insertion ;
un deuxième endoscope (1B) qui partage la même unité de source de lumière (42) ou processeur vidéo (43) avec le premier endoscope (1A) et dont la section souple (45) présente sensiblement le même diamètre externe sur la totalité de sa longueur, la section souple (45) étant contenue dans l'unité d'insertion ; et
un troisième endoscope (1C) qui partage la même unité de source de lumière (42) ou processeur vidéo (43) avec le premier endoscope (1A) et le deuxième endoscope (1B) et dont la section souple (46) présente sensiblement le même diamètre externe sur la totalité de sa longueur et présente un diamètre externe plus petit que le diamètre externe de la section souple (45) du deuxième endoscope (1B), la section souple (46) étant prévue dans l'unité d'insertion, dans lequel :
le diamètre externe de la partie de grand diamètre (10b) de la section souple (10) du premier endoscope (1A) est sensiblement identique ou inférieur au diamètre externe de la section souple (45) du deuxième endoscope (1B) et est plus grand que le diamètre externe de la section souple (46) du troisième endoscope (1C), et
le diamètre externe de la partie de petit diamètre (10a) de la section souple (10) du premier endoscope (1A) est sensiblement identique au diamètre externe de la section souple (46) du troisième endoscope (1C).

2. Système (41) d'endoscope selon la revendication 1, dans lequel le diamètre externe de la partie de petit diamètre (10a) du premier endoscope (1A) est supérieur au diamètre externe de la section souple (46) du troisième endoscope (1C).

3. Système (41) d'endoscope selon la revendication 1 ou 2, dans lequel le diamètre externe de la partie de grand diamètre (10b) du premier endoscope (1A) est sensiblement identique au diamètre externe de la section souple (45) du deuxième endoscope (1B).

4. Système (41) d'endoscope selon l'une quelconque des revendications 1 à 3, dans lequel la section souple (10) du premier endoscope (1A) comprend en outre une partie conique (10c) reliant la partie de petit diamètre (10a) et la partie de grand diamètre (10b).
